Europäisches Patentamt

**European Patent Office**

**Office européen des brevets**

(11) Publication number: **0 153 003**
A2

# EUROPEAN PATENT APPLICATION

(21) Application number: **85300186.5**

(22) Date of filing: **11.01.85**

(51) Int. Cl.⁴: **A 61 K 31/365,** A 61 K 47/00, A 61 K 31/165

(30) Priority: **01.02.84 GB 8402574**

(43) Date of publication of application: **28.08.85** **Bulletin 85/35**

(84) Designated Contracting States: **BE CH DE FR GB IT LI LU NL SE**

(71) Applicant: **FISONS plc, Fison House Princes Street, Ipswich Suffolk IP1 1QH (GB)**

(72) Inventor: **Ahmed, Maqbool, 104 Rokeby Park, Hull HU4 7QF (GB)**
Inventor: **Stevenson, Neil Arthur, 16 Longcliffe Gardens Nanpantan, Loughborough Leicestershire (GB)**

(74) Representative: **Craig, Christopher Bradberry et al, Fisons plc 12 Derby Road, Loughborough Leicestershire LE11 0BB (GB)**

(54) **Pharmaceutical formulations containing sodium cromoglycate.**

(57) There is described a suspension of finely divided sodium cromoglycate in an oily non-aqueous vehicle. The oily vehicle is preferably structured and may also contain another active ingredient, e.g. an antibiotic.

The compositions are useful in the treatment of conditions of the eye or nose in which conditions allergy or immune reactions play a contributory part.

EP 0 153 003 A2

- 1 -

PHARMACEUTICAL FORMULATIONS CONTAINING SODIUM CROMOGLYCATE

This invention relates to a novel formulation of sodium cromoglycate.

Aqueous formulations of sodium cromoglycate are known, but suffer from the disadvantage that they will support bacterial growth unless they contain appropriate preservatives. However, the presence of preservatives can be disadvantageous in that sensitive patients, and particularly allergic patients, may react adversely to the preservatives.

Various non-aqueous drug dispersions, e.g. of lipophilic steroids and antibiotics, are known. However, these dispersions either contain a very low proportion of drug, e.g. below about 0.5% w/w, or are such that on standing they settle. When the compositions settle, at best they need shaking before use, and at worst they will not redisperse evenly, thus leading to variability of dose.

We have found that a wide variety of oily vehicles on their own or admixed with so called supersolvents (e.g. propylene glycol, polyethylene glycol, ethyl acetate etc) do not dissolve sodium cromoglycate.

Structured aqueous vehicles are known for suspending drugs, but structured non-aqueous oily vehicles are not known, or are at least uncommon, in the pharmaceutical art.

Surprisingly we have now found that the strongly

- 2 -

hydrophilic drug sodium cromoglycate, in finely divided, e.g. micronised form, can be formulated as a dispersion in certain vehicles, that these dispersions can be stable at relatively high concentrations, and that they do not need the presence of preservatives to maintain sterility.

According to the invention we provide a suspension of finely divided sodium cromoglycate in an oily non-aqueous vehicle.

The suspension preferably contains 0.5% to 20%, more preferably 0.5% to 5%, and especially 2.0 to 4.0%, w/w of sodium cromoglycate. The suspension may also contain one or more other active ingredients in addition to the sodium cromoglycate, e.g. an antibiotic such as chloramphenicol. The proportion of other active ingredient will vary according to the particular active ingredient and its biological activity; we have however found that from 0.1 to 1.0%, e.g. 0.5% w/w of an antibiotic, such as chloramphenicol is suitable. We prefer the proportion of antibiotic to sodium cromoglycate to be in the range 1:50 to 1:0.5 w/w.

The sodium cromoglycate (and any other active ingredient which is not soluble in the oily vehicle) is preferably in a form having a mass median diameter of less than 20, and preferably less than 10, microns. We prefer that not less than 90% by weight of the particles are less than 10 microns in size and that not less than 50% by weight of the particles lie within the range 2 to 6 microns. Aggregates of the finely divided sodium cromoglycate may be present, but we prefer that no individual particle or aggregate be of greater than 90 microns in diameter and that most aggregates are of less than 30 microns in diameter. The presence of individual particles, or aggregates which cannot easily be broken down by blinking of the eye, of greater than 90 microns in diameter, will tend to cause a gritty feeling in the eye. The individual particles used to make up the suspension are preferably strong so that further uncontrolled physical degradation does not take place during the manufacture of the suspension.

The sodium cromoglycate preferably contains less than 8%, and preferably from 4.5 to 7.0%, w/w of water. This water is tightly bound by the sodium cromoglycate and is not available in the vehicle to encourage microbial growth. We prefer the composition to contain less than 0.63 available water (Aw).

The oily vehicle is preferably structured, e.g. by a non-metallic pharmaceutically acceptable structuring agent.

The oily vehicle should also be stable and pharmaceutically acceptable, especially for application to the eye. The vehicle is preferably free of antigenic

- 4 -

properties, and is also preferably a good suspending agent for the finely divided sodium cromoglycate, e.g. is of about the same density as the finely divided sodium cromoglycate. The oily vehicle is preferably substantially anhydrous and also is preferably such that the sodium cromoglycate is substantially insoluble therein. The viscosity of the suspension is desirably such that it will flow freely at ambient temperatures and will be retained in the eye or nose for a reasonable time. Suitable viscosities are in the range 2 to 500 centipoise.

By a structured oily vehicle we mean an oily liquid containing one or more substances which, because of their molecular physical characteristics and method of manipulation, associate in the vehicle in a pattern. This pattern can be represented as a 3-dimensional network or sponge. The degree of disarrangement in the pattern will depend upon the shape of the molecules, e.g. planar and charged, or long fibres.

To obtain the required arrangement special techniques, e.g. flash cooling, may be required.

The structure is intended to entrap the drug particles thereby slowing down their sedimentation or even appearing to prevent it altogether. These structured vehicles exhibit non Newtonian properties. Thus typically they have a yield point and exhibit pseudoplastic flow. When subjected to shear the vehicle generally shows an initial higher viscosity which is followed by a break in the profile followed by a lowering of the viscosity. This change may or may not be isothermally reversible.

The oily vehicle may be, for example, a fatty acid ester, e.g. a triglyceride ester of a mixture of vegetable (e.g. coconut and palm kernel) fatty acids of medium chain length ($C_8$-$C_{10}$) and succinic acid, and having a mean molecular weight of from about 600 to 700. The fatty acid ester may be structured by incorporation of from about 0.2 to 2.0, e.g. about 0.75, % w/w of 'Thixcin R' (an organic derivative of castor oil having a melting point of about $86^{o}C$).

Alternatively, the oily base may be a mineral oil which may be structured by incorporation of a polyethylene. The mineral oil preferably contains from about 0.4 to 1.5% w/w, e.g. 0.5% or 1.0% w/w, of the polyethylene. The polyethylene preferably has a molecular weight of from 19,000 to 23,000, e.g. of about 21,000. The mineral oil is preferably a heavy mineral oil of from 300 to 380, e.g. about 340, second Saybolt viscosity. The structured nature of the polyethylene in the mineral oil may be formed by shock cooling of an appropriate solution, e.g. at a rate of about $10^{o}C$ per

. second.

The suspensions of the invention may be made by dispersing the finely divided sodium cromoglycate in the oily vehicle. The vehicle may, when it is a solvent for any other active ingredients, have those active ingredients dissolved in it before the sodium cromoglycate is dispersed. The vehicle may also be in its structured form before the dispersion of the sodium cromoglycate, or the structure may be formed, e.g. by cooling, after the dispersion has been made.

According to the invention there is also provided a method of treatment of conditions of the eye or nose, in which conditions allergy or immune reactions play a contributory part which method comprises administration of a suspension according to the invention topically to a patient having such a condition.

The dosage to be administered will of course vary with the condition to be treated, with its severity and with its location. However, in general for use in the eye a dosage of about 1 or 2 drops (e.g. from 0.66 to 1.32mg of active ingredient) into the affected eye from 1 to 4 times a day is found to be satisfactory. More frequent dosage may, of course, be used if desired. For use in the nose a dosage of 1 or 2 drops (e.g. about 0.66mg to 1.32mg of active ingredient) is indicated per nostril.

Conditions of the outer eye in which the method of the invention may be used include allergic rhinitis, vernal catarrh (vernal kerato-conjunctivitis) and marginal corneal ulceration or infiltration. Other conditions which may be treated by the method of the invention include the occular effects of hay fever, 'allergic eyes' where the allergen is known or unknown and spring/summer conjunctivitis. This latter term is used to mean allergic disorders of the eyes occurring in the spring and summer where an external allergen plays a part in the disorder. Further conditions of the eye which may be mentioned are 'irritable eye' or 'non-specific conjunctivitis', Herpes Simplex Keratitis and Conjunctivitis, Herpes Zoster Keratitis and Conjunctivitis, adenovirus infections, phlyctenular conjunctivitis, corneal homograft rejection. Trachoma, anterior uveitis, blepharitis and drug sensitivity.

When the suspension also contains an antibiotic, e.g. chloramphenicol, the suspension may further be used to treat bacterial infections, e.g. of the eye. We have surprisingly found that the presence of the sodium cromoglycate can relieve or alleviate the discomfort and pain of the infection more rapidly than when the antibiotic is present on its own.

- 8 -

Conditions of the nose which may be mentioned include seasonal rhinitis, e.g. hay fever; perennial rhinitis, nasal polyps and allergic manifestations of the nasopharynx.

The suspensions may be put up in single application containers containing from 0.3 to 0.7ml suspension or in multi-dose (plastics, e.g. polyethylene, or glass) packs containing 5 to 20, preferably 7.5 or 17.5ml of suspension.

The invention is illustrated, but in no way limited, by the following Examples:-

Example 1

| | |
|---|---|
| Chloramphenicol base BP (micronised - mean particle size 2 to 8 microns not less than 99% by weight less than 20 microns) | 10g |
| Sodium cromoglycate (micronised) | 40g* |
| Plastibase 5W (Plastibase is a | to 2000g |
| Trade Mark) | |

*Expressed as anhydrous material.

The chloramphenicol and the sodium cromoglycate are suspended in 400g of the Plastibase 5W and the resulting suspension is then dispersed in the remaining Plastibase 5W using a Silverson mixer to produce a mobile oily suspension.

The formulation may be made under aseptic conditions using sterile constituents.

- 9 -

The resulting dispersion exhibits pseudoplastic behaviour and has a yield point.   Upon microscopic examination the dispersion was found to have substantially no aggregate of greater than 30 microns.

Upon storage at room temperature for 12 months no sodium cromoglycate sedimented out.

Plastibase 5W is a neutral, non-irritating and non-sensitising polyethylene-mineral oil formulation having a consistent structure.   This polyethylene-mineral oil structured material is stable, chemically inert, neutral and maintains rheological consistency over a wide range of temperatures (-15$^{\circ}$ to 60$^{\circ}$C).

The above formulation may be made in exactly the same manner, but omitting the chloramphenicol.

Example 2

| | |
|---|---|
| Chloramphenicol Base BP | 10g |
| Sodium cromoglycate BP (micronised) | 40g* |
| Thixcin-R (Thixcin is a Trade Mark) | 15g |
| Miglyol 829 Neutral Oil | to 2000g |
| (Miglyol is a Trade Mark) | |

*Expressed as the anhydrous weight

The chloramphenicol base was dissolved in hot (100$^{\circ}$C) Miglyol 829, followed by the dispersion of sodium cromoglycate (micronised) in the hot oil using a Silverson mixer.   The mixture was allowed to cool to 57°C. Thixcin-R was then added and the mixture was stirred thoroughly using the Silverson mixer. A structure was formed on cooling the formulation to 25°C.

The base may be sterilised by dry heat and the formulation made under aseptic conditions.

Miglyol 829 neutral oil is a triglyceride mixture of saturated fatty acids of vegetable origin with medium chain length ($C_8$-$C_{10}$) crosslinked with succinic acid. It is a clear yellowish viscid oil, neutral in taste and smell. It is biologically degradable; has a dynamic viscosity of 230-260 mPa.s (mN m$^{-2}$s) at 20°C and is immiscible in water.

Thixcin-R is an organic derivative of castor oil with a melting point of 86°C. It is a white, finely divided, non-discolouring, and non-hygroscopic powder.

Microscopically, the formulation was uniformly dispersed with substantially no aggregates of greater than 30 microns. The formulation is thixotropic and exhibits pseudoplastic behaviour. After two months' storage at room temperature and at 37°C the formulation was physically stable with sodium cromoglycate in suspension.

8745H(ir)/jaa

- 11 -

What we claim is:-

1. A suspension of finely divided sodium cromoglycate in an oily non-aqueous vehicle.

2. A suspension according to Claim 1 containing 0.5% to 5% w/w of sodium cromoglycate.

3. A supension according to Claim 1 or 2, wherein the sodium cromoglycate has a mass median diameter of less than 20 microns.

4. A suspension according to any one of the preceding claims, which contains an antibiotic.

5. A suspension according to Claim 4, containing from 0.1 to 1.0% w/w of chloramphenicol.

6. A suspension according to Claim 4 or 5, wherein the ratio of antibiotic to sodium cromoglycate is in the range from 1:50 to 1:0.5 w/w.

7. A suspension according to any one of the preceding claims, wherein the oily vehicle is structured.

8. A suspension according to any one of the preceding claims, wherein the oily vehicle comprises a mineral oil.

9. A suspension according to Claim 8, wherein the mineral oil is a heavy mineral oil of from 300 to 380 second Saybolt viscosity and comprises 0.4 to 1.5% w/w of polyethylene.

10. A suspension according to Claim 9, wherein the polyethylene has a molecular weight of from 19,000 to 23,000.

8854H/jaa